(19) Europäisches Patentamt
European Patent Office
Office européen des brevets

(11) **EP 3 789 930 A1**

(12) **EUROPEAN PATENT APPLICATION**

(43) Date of publication:
**10.03.2021 Bulletin 2021/10**

(21) Application number: **19195868.5**

(22) Date of filing: **06.09.2019**

(51) Int Cl.:
*G06N 3/08* (2006.01)          *G06N 3/04* (2006.01)
*A61B 6/00* (2006.01)          *G06N 20/10* (2019.01)
*G06T 7/00* (2017.01)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Universität Bern
3012 Bern (CH)**

(72) Inventors:
• **Márquez Neila, Pablo
  1007 Lausanne (CH)**
• **Sznitman, Raphael
  1005 Lausanne (CH)**

(74) Representative: **Vesterinen, Jussi Tapio
  LUMI IP GmbH
  Rodtmattstrasse 45
  3014 Bern (CH)**

(54) **METHOD AND SYSTEM FOR DATA VALIDATION**

(57)     The present invention concerns a method for validating a test signal (13). The method comprises: initialising weight parameters of an artificial neural network (7); generating training signal feature vectors from training signals (11), a respective training signal feature vector characterising a respective training signal; defining a probability density function for the artificial neural network (7) by using the generated training signal feature vectors; training the artificial neural network (7) by using the probability density function so that during training, new training signal feature vectors are generated that are iteratively moved towards higher feature vector density regions in a feature vector space as the training progresses for obtaining trained weight parameters of the artificial neural network as trained and a probability density function as trained. Once the network has been trained, then the method comprises generating a test signal feature vector for the test signal (13) by using the trained weight parameters of the artificial neural network as trained; computing a first value of the probability density function as trained for the test signal feature vector, the computed first value indicating a density of the training signal feature vectors in the feature vector space at a test signal feature vector location in the feature vector space; and using the first value to determine whether or not the test signal is valid.

**Fig. 1**

**Description**

FIELD OF THE INVENTION

The present invention relates to a method of carrying out a data validation process by using an artificial neural network in order to detect invalid signals. The present invention also relates to a computer program product comprising instructions for implementing the steps of the method, as well as to a data validation apparatus configured to carry out the proposed method.

BACKGROUND OF THE INVENTION

Data validation is the process of ensuring that the input to a data processing pipeline is correct and useful. It is a critical part of software systems running in production. Image processing systems are no different, whereby problems with data acquisition, file corruption or data transmission, may lead to a wide range of unexpected issues in the acquired images. Until now, most image processing systems or imaging systems of this type involved a human in the loop that could detect these errors before further processing. But with the advent of powerful deep learning methods, tools for imaging systems, such as medical image processing systems, are becoming increasingly autonomous and can go from data acquisition to final data analysis or medical diagnosis without any human interaction. However, deep networks are known for their inability to detect corruption or errors in the input data.

Over the last decade, the number of acquired medical image scans has steadily grown and represents today over 90% of all medical data in hospitals world-wide. Driven by widespread access to imaging devices, automated methods dedicated to processing medical scans are becoming increasingly critical to cope with the scale of produced images. Naturally, machine and deep learning play key roles in filling this need. At the same time, it is well known that deep networks have very limited extrapolation capacities and that properly trained deep networks behave unpredictably when presented with data that lie outside of their training data distribution. Given that acquisition devices, data transmission systems and other software systems can all fail in unexpected ways and lead to corrupted images or data sets more broadly, any automatic medical diagnosis from such images should be prevented in fear of providing inaccurate results, if not, dangerously misleading ones. The fundamental problem however is that it is not easy to know beforehand how and when a device will produce corrupted images or data sets more broadly.

Despite its importance, the problem of image data validation has been overlooked in many imaging fields and in particular medical imaging community. On the other hand, a great deal of research has focused on anomaly detection in medical imaging. In particular, previous methods have mainly focused on identifying abnormal or pathological structures using statistical shape analysis, clustering and one-class learning. However, none of these works are valid for detecting image corruption. A paper by Ruff, L., Vandermeulen, R., Goernitz, N., Deecke, L., Siddiqui, S.A., Binder, A., Muller, E., Kloft, M.: Deep one-class classification, In: ICML. vol. 80, pp. 4393-4402 (2018), discloses a one-class deep learning method, where a neural network learns a low-dimensional ball to model the data and rejects outliers that fall outside of this ball. Autoencoders also allow data distributions to be learnt, but typically only do so well for small images or patches, making them ill-suited for large medical images. Generative models, such as generative adversarial networks (GANs) or flow-based methods may be able to learn distributions of high-resolution images, but it has been found out that these methods in fact perform poorly on medical images, for instance.

To date, the main approach to detect when data content differs from its intended content is to perform a battery of tests on the data in order to evaluate its integrity. Unfortunately, each manufacturer and device need their own set of rules. This is both time consuming and costly for manufacturers. At the same time, there is still no guarantee that the tests developed and implemented by a manufacturer will catch all potential corruptions that could appear when the device is used in practice.

BRIEF DESCRIPTION OF THE INVENTION

The present invention aims to overcome at least some of the above-identified disadvantages of data validation solutions. More specifically, the present invention aims to provide an improved deep data validation solution, which may be used in various fields, such as in the imaging field, and in particular in the medical imaging field.

According to a first aspect of the invention, there is provided a method for validating a test signal as recited in claim 1.

The present invention thus proposes a deep validation method that learns to measure how correct a given test signal, such as an image, is, and is able to raise human attention when the input test signal contains high levels of noise, unexpected occlusions, or corruption of any other kind. The proposed method is precise and is able to detect corrupted signals reliably. Furthermore, the proposed method is very general and is easily adaptable to new machines and machine learning methods. The proposed solution can be distributed as a piece of software (library or cloud service). Customers can integrate it within their trainable data processing systems, such as imaging systems. The proposed solution can be incorporated into any device manufacturer's data processing pipeline that wants to use machine learning technology.

It is to be noted that image quality assessment (IQA) methods also aim to detect artefacts in images. However, the concept of quality in these methods is traditionally connected to the subjective human perception of images. According to the present invention, the quality is related to the performance of a trained system, which may be an imaging system. In other words, the proposed method does not mind if the visual quality of a given image is poor as long as it can guarantee that the image is suitable for subsequent processing. While IQA is a part of the quality of customer's experience (QoE), the proposed method falls in the category of data validation.

According to a second aspect of the invention, there is provided a computer program product comprising instructions for implementing the steps of the method according to the first aspect of the present invention when loaded and run on computing means of an electronic device.

According to a third aspect of the invention, there is a data validation apparatus for implementing the method according to the first aspect of the present invention.

Other aspects of the invention are recited in the dependent claims attached hereto.

BRIEF DESCRIPTION OF THE DRAWINGS

The invention will now be described in more detail with reference to the attached drawings, in which:

- Figure 1 is a simplified block diagram showing an example imaging system according to the present invention;

- Figures 2 and 3 show example feature vector distributions of training signals in a feature vector space; and

- Figures 4A and 4B show a flow chart illustrating an example data validation method according to the present invention.

DETAILED DESCRIPTION OF THE INVENTION

It should be noted that the figures are provided merely as an aid to understanding the principles underlying the invention, and should not be taken as limiting the scope of protection sought. Where the same reference numbers are used in different figures, these are intended to indicate similar or corresponding features. It should not be assumed, however, that the use of different reference numbers is intended to indicate any particular degree of difference between the features to which they refer. The present invention will be explained in the context of a data validation process for medical images, such as medical image scans. However, it is to be noted that teachings of the invention are by no means restricted to image data validation or to medical applications. More generally, the teachings of the invention can be applied to validating D-dimensional, finite length signals, where D is any integer from 1 to 4. If the teachings of the present invention are applied to imaging solutions, then the present invention may be applied to any imaging system, where it would be beneficial to carry out a data validation step prior to further analysing or processing the images by using e.g. an artificial neural network.

The present invention proposes a data signal validation method that learns to recognise the validity or viability of data signals from a given system, in this example a medical system. The method is then configured to determine whether or not a given data signal is appropriate for further processing or evaluation. As soon as the present method detects a signal outside of the learnt data, either by noise, occlusion or any other perturbation, it interrupts the analysis and optionally raises the attention of a human operator for further inspection. Furthermore, for example in critical systems with software redundancy (i.e., having multiple software systems from different developers performing the same tasks) or with sensor redundancy, the proposed data validation method could disable the module that generates invalid signals, or the software system that processes those signals. This could be done in an automatic manner with no human interaction. The proposed method allows validating incoming signals, such as data elements or items of data, meant to be processed by a method which the operator knows nothing about except the data or signals which it was trained on. To this end, the present invention introduces a deep data validation (DDV) framework for signals, in the example below for images typically acquired by medical devices. It is proposed that every trainable model, such as a deep network, trained in a supervised manner for a given task, be augmented with a DDV model that explicitly learns the training data distribution and rejects signals that do not belong to it at test time. Be it for differential diagnosis or segmentation methods, preferably each test time signal can be validated with a DDV model first and only then analysed by the intended method if approved by the DDV model. As such, the contributions of the present invention are (1) introducing the concept of data validation for signals, such as high-resolution medical images, and (2) proposing a non-parametric solution that is easy to train, stable and fast at test time. Experimental assessment of the proposed method has shown that the performance of the proposed method outperforms existing baselines by a large margin when applied e.g. to various medical datasets.

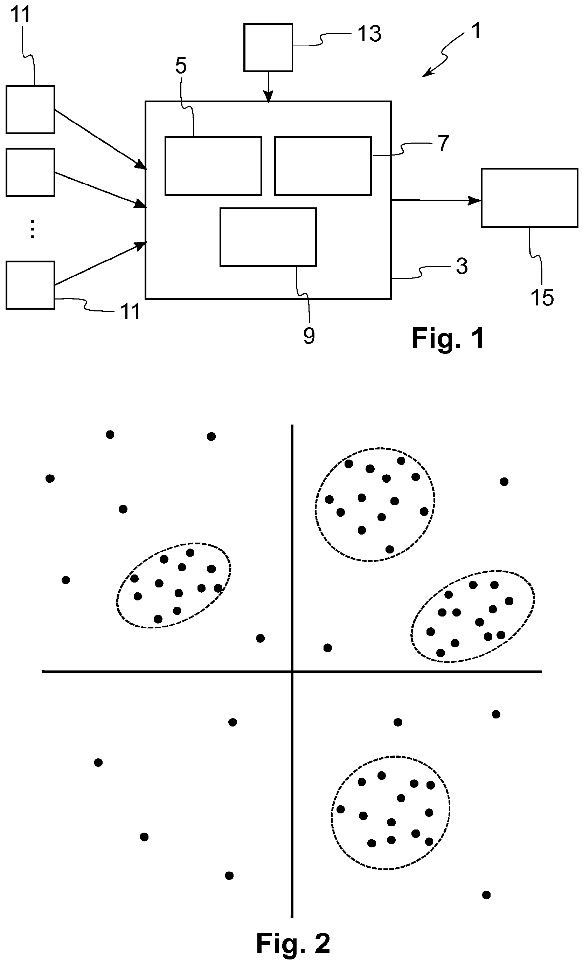

Figure 1 schematically illustrates some functional or physical elements that are useful for understanding the teachings of the present invention. The present nonlimiting embodiment is explained in the context of a medical imaging system 1 configured to process medical images. The example imaging system shown in Figure 1 comprises a data validation element 3, module or unit, hereinafter referred to as a DDV module. The DDV module 3 comprises a training and evaluation processor 5, which is configured to run a training algorithm for training an artificial neural network 7, which in this example is part of the DDV module 3. The DDV also comprises a probability density function 9 which is used to train the artificial neural network. The probability density function is also learnt from the output of the artificial neural network, and the probability density function is used at test time to estimate the validity of an image. The DDV 3 may also comprise a data storage unit serving as memory and/or storage for the DDV module 3. As explained later in more detail, during a training phase of the neural network 7, a training data set comprising a set of training images 11 is fed into the DDV module 3 to train the artificial neural network 7. More specifically, during this phase the training images are advantageously fed into the training unit numerous times to iteratively determine parameters of the artificial neural network 7, and in particular weights of the artificial neural network 7. During a test phase, a test image 13 to be analysed is fed into the trained DDV module 3 to determine whether or not the test image is valid, and optionally to determine how valid or corrupted the test image is. If the test image is determined to be valid, then the test image can be forwarded to a processing circuit 15 for further analysis or processing. The training images or signals advantageously have the same length, size or dimension as the test images or signals.

Problem formulation

In the present description, it is assumed that a task-specific algorithm running on the processing circuit 15 has been trained with a dataset

$$\mathcal{X} = \{\boldsymbol{x}^{(i)}\}_{i=1}^{N},$$

where $x \in \mathcal{J}$ are images 11. For example, this may be a segmentation method tasked to outline brain tumours in magnetic resonance imaging (MRI) and trained on a large dataset of examples. Given this, the aim is to learn a function that can identify corrupted images at test time, with the objective of stopping any subsequent method from processing them.

In particular, the aim is to train a discriminative model, e.g., a deep network or the artificial neural network 7 in this example, to learn the probability that a given input image 13 is corrupted. Training such a model would be easy if it were possible to acquire examples from the distribution of corrupted and valid images, $\mathcal{D}^+$ and $\mathcal{D}^-$, respectively. Unfortunately, the distribution of corrupted images $\mathcal{D}^+$ is extremely complex, containing modified versions of images in $\mathcal{D}^-$ with wide ranges of possible corruptions (e.g., noise, occlusions, missing pixels, different zoom, alignment problems), as well as images that are completely unrelated to $\mathcal{D}^-$. For instance, if $\mathcal{D}^-$ is the distribution of chest X-rays, elements in $\mathcal{D}^+$ would also include computed tomography (CT) scans, MRIs and other real-world photographs. In a sense, $\mathcal{D}^+$ is the complementary distribution of $\mathcal{D}^-$ in the space of all possible images. Therefore, the idea of obtaining a representative set of samples from $\mathcal{D}^+$ is simply hopeless.

To overcome this, the problem is framed in the context of one-class (images in the same category) learning, where it is assumed that $\mathcal{X}$ (the training dataset) is representative of the distribution of valid images $\mathcal{D}^-$ The aim then is to learn a score or metric that measures the corruption level of a given image 13.

One approach would be to estimate the value of the probability density function, $p(x)$ of $\mathcal{D}^-$ in the image space $\mathcal{J}$ itself. However, because medical images are typically high dimensional images (e.g., 1000×1000 pixels for a chest X-ray), doing so is difficult in practice. Taking this into consideration, the present approach instead proposes to learn a projection from $\mathcal{J}$ to a manageable lower-dimensional latent space or feature vector space using a deep network, $f : \mathcal{J} \rightarrow \mathbb{R}^d$. The function $f$ thus defines the operation of the artificial neural network 7 by means of weight parameters of the artificial neural network. In this projected space, we now define the probability density function at a feature vector location $\boldsymbol{z}$ in the feature vector space as $p(\boldsymbol{z})$, where $\boldsymbol{z} = f(\boldsymbol{x})$ corresponds to the latent vector or feature vector of an image. In this example a respective feature vector is a sequence of numbers, for instance (-3, 4) in a very simplified example, where the feature vector space is only two-dimensional, charactering a respective image. Thus, the number of items in a given feature vector equals the dimension of the feature vector space. The dimension of the feature vector space may be between 2 and 64, or more specifically between 8 and 64 or between 10 and 40. It has been discovered that dimensions 16 and 32 perform particularly well in the present context. Since we cannot assume any specific distribution of the data in the feature vector space, a kernel density estimation is used:

$$p(\boldsymbol{z}) = \frac{1}{N}\sum_{\boldsymbol{x}^{(j)}\in\mathcal{X}} K_h\left(\boldsymbol{z} - f\big(\boldsymbol{x}^{(j)}\big)\right), \tag{1}$$

where $K_h \propto e^{-\frac{\|z\|^2}{2h^2}}$ is the Gaussian kernel with bandwidth $h \in \mathbb{R}^+$. For simplicity of notation, it is assumed that $\int_{\mathbb{R}^d} K_h(\boldsymbol{z})d\boldsymbol{z} = 1.$ It is to be noted $f(\boldsymbol{x}^{(j)})$ is the feature vector for image or more specifically training image $j$. As such, we represent our valid feature vector data distribution using a non-parametric representation. The probability density function $p$, which takes a feature vector $\boldsymbol{z}$ as an input, thus gives a density of feature vectors for any given point $\boldsymbol{z}$ in the feature vector space.

Training

To train the proposed DDV module 3, we maximise the log-likelihood of the feature vectors of $\mathcal{X}$ ,

$$\arg\max_f \log \prod_{\boldsymbol{x}^{(i)}\in\mathcal{X}} p\left(f\big(\boldsymbol{x}^{(i)}\big)\right), \tag{2}$$

which may be done using any standard stochastic-gradient-based method with minibatches, for example as explained in "Kingma, D.P., Ba, J.: Adam: A method for stochastic optimization, ICLR (2014)". During training, Equation 2 is repeatedly computed once per minibatch for all the training images belonging to the respective minibatch. An epoch is here understood to mean the iterations required to go through the training data set during training. A minibatch is here understood to comprise a subset of the training images, i.e. a subset of $\mathcal{X}$ . However, solving Equation 2 is computationally challenging because the network $f$ is used twice: once to define the probability density function $p$ (relating to term $f(\boldsymbol{x}^{(j)})$ in Equation 3) and once to compute the image feature vectors from the minibatches (relating to term $f(\boldsymbol{x}^{(i)})$ in Equation 3). Equation 3 below is obtained by combining Equations 1 and 2:

$$\arg\max_f \sum_{\boldsymbol{x}^{(i)}\in\mathcal{X}} \log \sum_{\boldsymbol{x}^{(j)}\in\mathcal{X}} K_h\left(f\big(\boldsymbol{x}^{(i)}\big) - f\big(\boldsymbol{x}^{(j)}\big)\right) - N\log N. \tag{3}$$

Hence optimising over both $f$ at the same time may lead to instability issues, as we are updating $f$ to find the modes of the distribution $p$ and simultaneously updating the distribution $p$. To avoid such instabilities, we freeze $p$, i.e. the term $f(\boldsymbol{x}^{(j)})$, by keeping a constant copy of the network, $\overline{f}$ (corresponding to frozen $f(\boldsymbol{x}^{(j)})$), which is updated $\overline{f} \leftarrow f$ at every epoch during training. The fact of freezing $p$ leads to more stable training, but also allows us to build acceleration structures, such as kd-trees (to ignore some isolated feature vectors, which are far from other feature vectors), to efficiently compute the probability density function $p$ when the training dataset $\mathcal{X}$ is very large. Algorithm 1 summarises the proposed training procedure. The operator $\leftarrow\!\!\!/\,$ indicates a detached assignment that disables gradient computation by blocking back-propagation through it.

---

**Input:** Training dataset $\mathcal{X}$, network $f$ with parameters $w$, optimiser $\mathcal{O}$, max epochs

**Output:** Trained network $f$

    **for** epoch $\leftarrow$ 1 to max epochs **do**

        Create a frozen copy of the network: $\bar{f} \nleftarrow f$

        Compute the feature vectors of training dataset: $\mathcal{Z} \leftarrow \{\bar{f}(x^{(j)}): x^{(j)} \in \mathcal{X}\}$

        (Optional) Build a kd-tree with $\mathcal{Z}$ for efficient computation of $p$

        **for all** $\mathcal{M} \in$ minibatches of $\mathcal{X}$ **do**

            Compute log-likelihood of the minibatch: $\ell \leftarrow \sum_{x^{(i)} \in \mathcal{M}} \log p\big(f(x^{(i)})\big)$

            Back-propagate gradients: $\nabla_w \ell$

            Update weights: $w \leftarrow \mathcal{O}.\text{step}(w, -\nabla_w \ell)$

        **end for**

    **end for**

---

Algorithm 1.

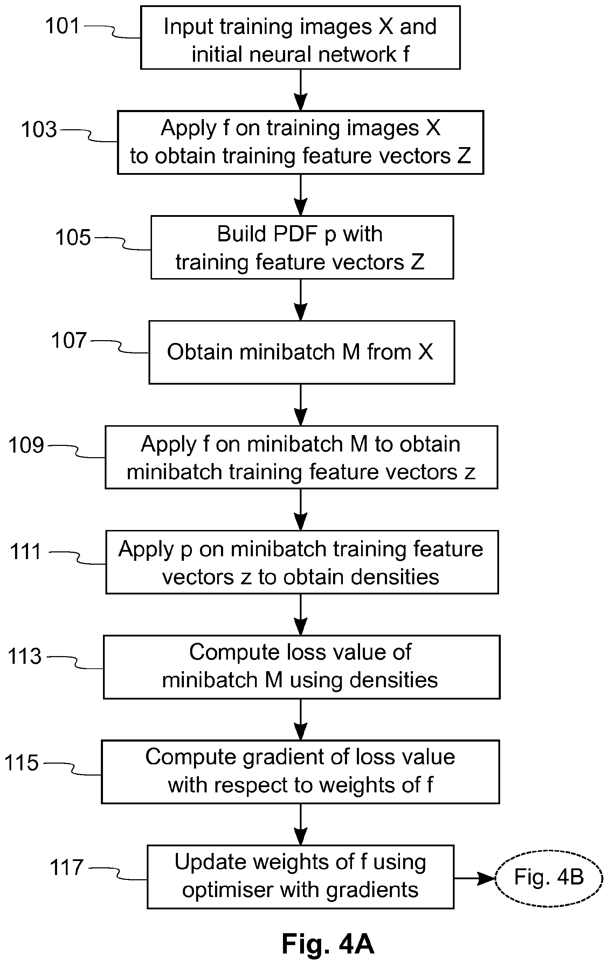

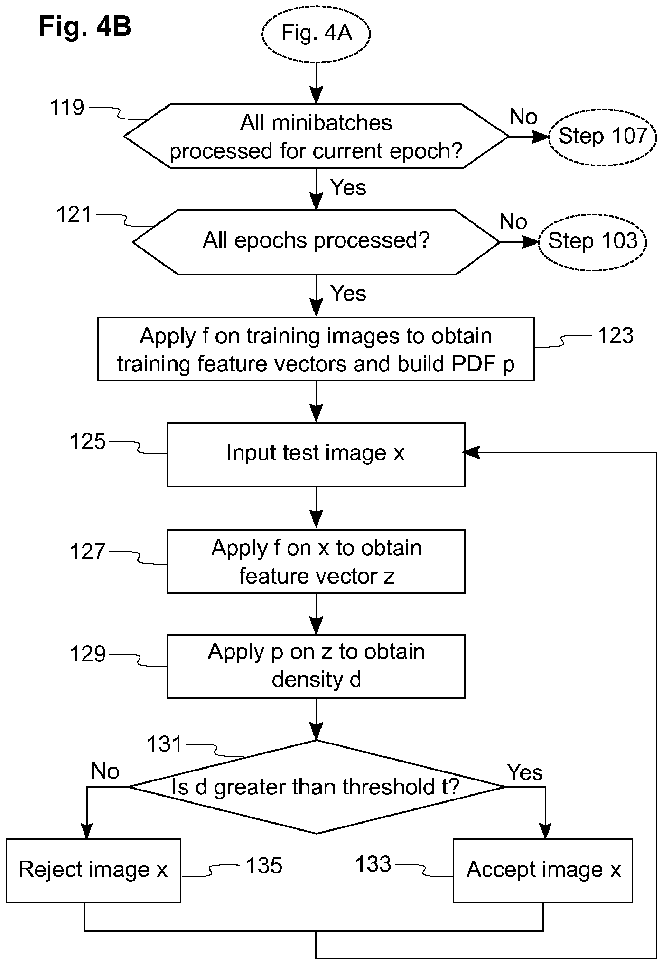

The number of epochs may be for instance between 1 and 100, or more specifically between 1 and 30, or between 2 and 10. Figure 2 shows in a very simplified manner an example feature vector distribution of the training images 11 belonging to the training data set $\mathcal{X}$. The distribution of Figure 2 is obtained by using the neural network 7 in its initial state, referred to as a state $\text{NN}_0$. At this stage, no weight parameters have been optimised yet. It can be seen that the density distribution includes one or more high-density regions or areas, where the density of the feature vectors in the feature vector space is relatively high. It is to be noted that during training, there is no need to select a fixed threshold for defining the high-density regions. The example distribution of Figure 4 has four high-density regions. The region(s) outside of the high-density regions is/are understood to be low-density region(s). Typically, most of the feature vectors lie within the high-density regions. The aim of the training phase is to optimise the weight parameters of the artificial neural network 7 so that as many as possible of the feature vectors of the training images 11 can be moved towards or into higher density regions as the training progresses so that after the training phase, the high-density regions indicate the locations of the feature vectors of correct or valid images.

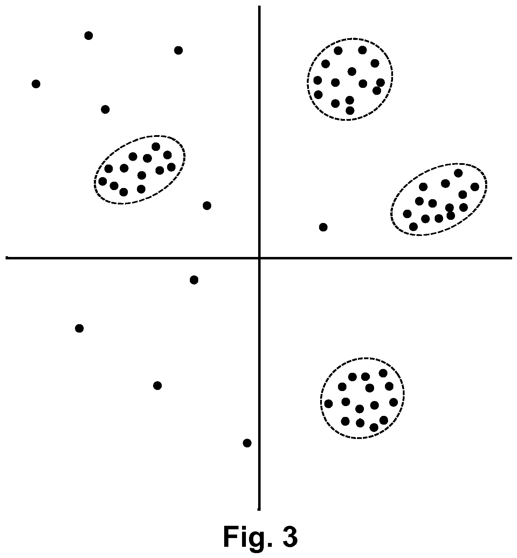

As soon as the first round of iteration has been completed, i.e. as soon as all the training images have been fed once into the artificial neural network 7 and thus after the first epoch, the values $f(x^{(j)})$ of the artificial neural network are updated. The state of the artificial neural network 7 can now be referred to as state $\text{NN}_1$. The training images are now fed again, either simultaneously or one after another, into the artificial neural network 7 to compute the feature vectors by using the latest or current weight parameters of the artificial neural network 7. Figure 3 shows a simplified example distribution of the feature vectors in the feature vector space after the second epoch. As can be seen, the sizes of the high-density regions have become smaller and more dense compared with the configuration of Figure 2. The location of one or more of the high-density regions may also change from one epoch to another. As was explained earlier, during a given epoch, the high-density region configurations or the probability density function remain(s) fixed in this example. They are only updated after each epoch. It is to be noted that during the training phase, the weight parameters of the artificial neural network are updated once per minibatch, which means that the values $f(x^{(i)})$ are updated once per minibatch when computing Equation 3, which is solved once per minibatch for the training images belonging to the respective minibatch to obtain a single score or number per minibatch. Once the iterations for state $\text{NN}_1$ have been completed, and the values of $f(x^{(i)})$ and $f(x^{(i)})$ are updated, state $\text{NN}_2$ is reached. The iterations are continued as long as the probability distribution function substantially changes (during the iterations) or as long as the maximum number of epochs has not been reached. At this moment, a threshold may be selected to define the high-density regions. Only one threshold is selected. The threshold may be set so that for example 1% of the training images lie outside of the high-density regions. In the present example, the threshold value is set by the operator experimentally. Once the training phase is complete, the artificial neural network 7 is ready to be used to analyse test images as explained next and in particular to compute a feature vector for a given test image.

Test time

To evaluate a new image at test time, we use the negative logarithm of the value of the probability density function at the location of the feature vector of image $x$ $s(x)$ = -log$p(f(x))$, as the metric to measure the corruption level of the input image $x$. However, it is not necessary to use log-likelihood, but instead, only the value of the probability density function $p(f(x))$ could be used in the evaluation. The log-likelihood is advantageously used to bring the results to a more understandable space as the obtained probability values can be very small. The value of the probability density function $p(f(x))$ gives the density of the training image feature vectors in the feature vector space at the location of the feature vector of image $x$. The value of $p(f(x))$ thus tells the operator how dense the feature vector space is around point or image $x$. In other words, $p(f(x))$ thus gives the likelihood of the feature vector of the test image being in one of the high-density regions. In the present example, the lower the value of $s(x)$ is, the better the test image can be concluded to be. To determine whether or not the test image is a valid image, the value of $s(x)$ is compared to the pre-defined threshold value. If the value of $s(x)$ is determined to be smaller than the threshold value, then according to the present example, the test image is determined to be valid. In other cases, the test image is determined to be invalid or corrupted.

The teachings of the above-described embodiment are summarised in the flow chart of Figures 4A and 4B. In step 101, the set of training images 11 of the training data set $\mathcal{X}$, and an initial model of the artificial neural network, or more specifically its initial weight parameters, are fed into the DDV module 3, and more specifically to the training algorithm running on the training and evaluation processor 5. This step may also comprise feeding the initial weight parameters into the artificial neural network to initialise it. In step 103, the training images are fed into the artificial neural network. In other words, the function $f$ (or the neural network $f$) is applied on the training images 11 for obtaining training feature vectors $Z$. In step 105, the probability density function (below and in the flow chart referred to as a PDF) $p$ is built or defined by using the training image feature vectors $Z$. Thus, in step 105, Equation 1 is used. In step 107, the next minibatch $\mathcal{M}$ from $\mathcal{X}$ is obtained. In step 109, the function $f$ of the artificial neural network is applied on the training images of the current minibatch for obtaining minibatch training image feature vectors $z$. In step 111, the function $p$ is applied on the minibatch training image feature vectors $z$. In other words, the values of the PDF $p$ are computed for every training image in the current minibatch for obtaining densities of the training image feature vectors in the feature vector space at a given training image feature vector location. In step 113, a loss value is computed for the current minibatch by using the densities obtained in step 111. In this step, a loss value is obtained for each training image in the minibatch and then these values are summed to obtain one loss value per minibatch. The loss function can be defined from Equation 3 as

$$-\log \sum\nolimits_{x^{(j)} \in \mathcal{X}} K_h \left( f\left(x^{(i)}\right) - f\left(x^{(j)}\right) \right) - N\log N.$$

The obtained loss value per minibatch indicates how low the density of the training vectors is for the current minibatch, i.e. how bad or good the prediction of the network is for the minibatch. Thus, the operation of step 113 is equivalent to Equation 3, but in step 113, the loss values is summed over the training images in the current minibatch as opposed to being summed over the entire training dataset. In step 115, the gradient of the loss value is computed with respect to the current weights of the artificial neural network. In step 117, the weights of the artificial neural network are updated by using an optimiser (such as the one in "Kingma, D.P., Ba, J.: Adam: A method for stochastic optimization, ICLR (2014)"), which uses the gradients. The optimiser can be considered to be a function having an internal memory. In other words, in this step, the loss value is minimised, or Equation 3 is maximised for the current minibatch.

In step 119, it is determined whether or not all the minibatches of the current epoch have been processed. If there are still at least one minibatch to be processed, then the process continues in step 107. If on the other hand all the minibatches have been processed, then in step 121 it is determined whether or not the maximum number of epochs has been reached. If there are still some epochs left, then the process continues in step 103. If on the other hand the maximum number of epochs has been reached, then the process continues in step 123. In step 123, the function $f$ is applied on the training images to obtain most up-to-date or current feature vectors. Furthermore, also in this step, the PDF $p$ is built by using the most up-to-date feature vectors. It is to be noted that the processing up to this point (i.e., the training phase) is carried out "offline", prior to integrating the DDV module 3 into an end user device, such as a medical device. The subsequent steps as explained below are carried out "online", while the DDV module 3 is already integrated or connected to the medical device.

In step 125, a new test image $x$ is fed into the DDV module 3. In step 127, the function $f$ is applied on the test image $x$ for obtaining a test image feature vector $z$. In step 129, the PDF $p$ is applied on the test image feature vector to obtain the value of the PDF $p$, in other words the density $d$ at the location of the test image feature vector in the feature vector space. Thus, in this step the PDF $p$ receives $z$ as an input to calculate the density. In step 131, this density $d$ is compared to threshold *t* to determine whether or not the test image can be determined to be valid. If the density value *d* is greater than the threshold value *t*, then the test image is considered to valid (step 133). This means that it is determined to be suitable for further processing or analysis. If the condition of step 131 is not fulfilled, then in step 135 the test image is rejected as an invalid image. It is to be noted that in the example of Figures 4A and 4B, the density *d* (first value) was directly compared to the threshold *t* instead of taking a negative logarithm of the density *d* (second value) as done in *s*(**x**).

The above embodiment was described in the context of medical image processing. However, the teachings of the present invention are applicable in various technical fields. Some possible applications are briefly explained next. Self-driving cars use deep networks to process signals of different nature they receive through multiple sensors. Data validation is required to detect sensor malfunction. It could also happen that a car finds itself in an environment or setup for which it has not been trained (for example, the training focuses on road environments, but the car is in a roadless environment). Those cases could be detected by the present data validation method in order to disable or limit the self-driving system. Video surveillance systems are becoming more autonomous. Detecting video streams that behave in a different manner than the ones used for training can be done with the proposed data validation method. It could also be used for detecting suspicious behaviours that have not been observed before. In speech recognition systems (for example to automatically generate subtitles for online videos), data validation can detect parts of the speech spoken in different languages compared to the languages recognised by the system, and therefore the proposed method can stop subtitle generation to avoid nonsensical text.

The above described method steps may be carried out by suitable circuits or circuitry. The terms "circuits" and "circuitry" refer to physical electronic components or modules (e.g. hardware), and any software and/or firmware ("code") that may configure the hardware, be executed by the hardware, and or otherwise be associated with the hardware. The circuits may thus be operable to carry out or they comprise means for carrying out the required method steps as described above.

As used herein, unless otherwise specified the use of the ordinal adjectives "first", "second", "third", etc., to describe a common object, merely indicate that different instances of like or different objects are being referred to, and are not intended to imply that the objects so described must be in a given sequence, either temporally, spatially, in ranking, or in any other manner.

While the invention has been illustrated and described in detail in the drawings and foregoing description, such illustration and description are to be considered illustrative or exemplary and not restrictive, the invention being not limited to the disclosed embodiment. Other embodiments and variants are understood, and can be achieved by those skilled in the art when carrying out the claimed invention, based on a study of the drawings, the disclosure and the appended claims.

In the claims, the word "comprising" does not exclude other elements or steps, and the indefinite article "a" or "an" does not exclude a plurality. The mere fact that different features are recited in mutually different dependent claims does not indicate that a combination of these features cannot be advantageously used. Any reference signs in the claims should not be construed as limiting the scope of the invention.

**Claims**

1. A computer implemented method of validating a test signal, where the test signal is a D-dimensional, finite length signal, D being any integer from 1 to 4, the method comprising:

   • initialising (101) weight parameters of an artificial neural network (7);
   • receiving training signals (11) and generating (103) training signal feature vectors from the training signals, a respective training signal feature vector characterising a respective training signal;
   • defining (105) a probability density function for the artificial neural network (7) by using the generated training signal feature vectors;
   • training (107, 109, 111, 113, 115, 117) the artificial neural network (7) by using the probability density function so that during training, new training signal feature vectors are generated that are iteratively moved towards higher feature vector density regions in a feature vector space as the training progresses for obtaining trained weight parameters of the artificial neural network as trained and a probability density function as trained;
   • generating (127) a test signal feature vector for the test signal by using the trained weight parameters of the artificial neural network as trained;
   • computing (129) a first value of the probability density function as trained for the test signal feature vector, the computed first value indicating a density of the training signal feature vectors in the feature vector space at a test signal feature vector location in the feature vector space; and
   • comparing (131) the first value, or a second value depending on the first value, to a threshold value for determining whether or not the test signal is valid.

2. A method according to claim 1, wherein the test signal (13) comprises a test image (13), and the training signals (11) comprise training images (11).

3. A method according to any one of the preceding claims, wherein the method further comprises repeatedly computing (111, 113, 115, 117), during the training, a value of the probability density function for a respective training signal feature vector such that weight parameters of the artificial neural network are adjusted as the training progresses so that the training signal feature vectors are moved towards the higher feature vector density regions in a feature vector space.

4. A method according to any one of the preceding claims, wherein the training signals (11) are comprised in a training data set comprising N training signals, N being a positive integer, wherein the training data set is divided into n minibatches, n being a positive integer smaller than N, and wherein the method further comprises changing weight parameters of the artificial neural network once per minibatch during the training.

5. A method according to claim 4, wherein the method further comprises carrying out the following steps for all the minibatches, during the training:

• computing (111) a value of the probability density function for each of the training signal feature vectors in a respective minibatch;
• computing (113) a loss value for the respective minibatch by using the values of the probability density function, the loss value indicating how dense the training signal feature vectors are in the feature vector space at a minibatch feature vector location; and
• minimising (115, 117) the loss value by updating weights of the artificial neural network.

6. A method according to claim 5, wherein a respective loss value is computed for all the training signal feature vectors in the respective minibatch, and the respective loss values are summed to obtain the loss value for the respective minibatch.

7. A method according to any one of the preceding claims, wherein the training signals (11) are comprised in a training data set comprising N training signals, N being a positive integer, wherein an epoch is defined as one full iteration round during which all the training signals (11) of the training data set are processed once by the artificial neural network during the training, and wherein the probability density function is updated once per epoch.

8. A method according to claim 7, wherein the probability density function is updated by using the most recent training signal feature vectors obtained from the artificial neural network using the most recent weight parameters.

9. A method according to any one of the preceding claims, wherein the training signals (11) and the test signal (13) have substantially the same signal length and/or dimension.

10. A method according to any one of the preceding claims, wherein the probability density function is defined as a function of a given feature vector $\boldsymbol{z}$ as $p(\boldsymbol{z}) = \frac{1}{N}\sum_{\boldsymbol{x}^{(j)}\in\mathcal{X}} K_h\left(\boldsymbol{z} - f\left(\boldsymbol{x}^{(j)}\right)\right),$ where $N$ denotes the number of training signals, $K_h$ denotes Gaussian kernel with bandwidth $h \in \mathbb{R}^+$, $f$ is a function characterising the artificial neural network, $\mathcal{X}$ denotes a training signal data set such that

$$\mathcal{X} = \{\boldsymbol{x}^{(j)}\}_{j=1}^{N},$$

where $\boldsymbol{x} \in \mathcal{I}$ are the training signals.

11. A method according to claim 10, wherein during the training, the following equation is solved to determine the trained weight parameters:

$$\arg\max_{f} \sum_{\boldsymbol{x}^{(i)} \in \mathcal{X}} \log \sum_{\boldsymbol{x}^{(j)} \in \mathcal{X}} K_h \left( f\left(\boldsymbol{x}^{(i)}\right) - f\left(\boldsymbol{x}^{(j)}\right) \right) - N\log N.$$

12. A method according to any one of the preceding claims, wherein the method further comprises sending the test signal to a data processing apparatus (15) for further analysis only if the test signal is determined to be valid.

13. A method according to any one of the preceding claims, wherein a dimension of the feature vector space is between 10 and 40.

14. A computer program product comprising instructions for implementing the steps of the method according to any one of the preceding claims when loaded and run on computer means of an electronic device.

15. A data validation apparatus (3) for validating a test signal, where the test signal is a D-dimensional, finite length signal, D being any integer from 1 to 4, the apparatus comprising means for:

- initialising weight parameters of an artificial neural network (7);
- receiving training signals (11) and generating training signal feature vectors from the training signals, a respective training signal feature vector characterising a respective training signal;
- defining a probability density function for the artificial neural network (7) by using the generated training signal feature vectors;
- training the artificial neural network (7) by using the probability density function so that during training, new training signal feature vectors are generated that are iteratively moved towards higher feature vector density regions in a feature vector space as the training progresses for obtaining trained weight parameters of the artificial neural network as trained and a probability density function as trained;
- generating a test signal feature vector for the test signal by using the trained weight parameters of the artificial neural network as trained;
- computing a first value of the probability density function as trained for the test signal feature vector, the computed first value indicating a density of the training signal feature vectors in the feature vector space at a test signal feature vector location in the feature vector space; and
- comparing the first value, or a second value depending on the first value, to a threshold value for determining whether or not the test signal is valid.

**Fig. 1**

**Fig. 2**

Fig. 3

101 — Input training images X and
initial neural network f

103 — Apply f on training images X
to obtain training feature vectors Z

105 — Build PDF p with
training feature vectors Z

107 — Obtain minibatch M from X

109 — Apply f on minibatch M to obtain
minibatch training feature vectors z

111 — Apply p on minibatch training feature
vectors z to obtain densities

113 — Compute loss value of
minibatch M using densities

115 — Compute gradient of loss value
with respect to weights of f

117 — Update weights of f using
optimiser with gradients → Fig. 4B

**Fig. 4A**

**Fig. 4B**

Fig. 4A

119 — All minibatches processed for current epoch? — No → Step 107

Yes

121 — All epochs processed? — No → Step 103

Yes

123 — Apply f on training images to obtain training feature vectors and build PDF p

125 — Input test image x

127 — Apply f on x to obtain feature vector z

129 — Apply p on z to obtain density d

131 — Is d greater than threshold t?

No → Reject image x — 135

Yes → Accept image x — 133

Europäisches
Patentamt

European
Patent Office

Office européen
des brevets

# EUROPEAN SEARCH REPORT

Application Number

EP 19 19 5868

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DAVIDE ABATI ET AL: "AND: Autoregressive Novelty Detectors", ARXIV.ORG, CORNELL UNIVERSITY LIBRARY, 201 OLIN LIBRARY CORNELL UNIVERSITY ITHACA, NY 14853, 4 July 2018 (2018-07-04), XP081244028, * page 1 - page 7 * | 1-15 | INV. G06N3/08 G06N3/04 A61B6/00 G06N20/10 G06T7/00 |
| A | CAO VAN LOI ET AL: "A Hybrid Autoencoder and Density Estimation Model for Anomaly Detection", 31 August 2016 (2016-08-31), INTERNATIONAL CONFERENCE ON FINANCIAL CRYPTOGRAPHY AND DATA SECURITY; [LECTURE NOTES IN COMPUTER SCIENCE; LECT.NOTES COMPUTER], SPRINGER, BERLIN, HEIDELBERG, PAGE(S) 717 - 726, XP047354452, ISBN: 978-3-642-17318-9 [retrieved on 2016-08-31] * pages 1, 3 * | 1-15 | |
| A | WANG HONGZHI ET AL: "Progress in Outlier Detection Techniques: A Survey", IEEE ACCESS, vol. 7, 2 August 2019 (2019-08-02), pages 107964-108000, XP011740004, DOI: 10.1109/ACCESS.2019.2932769 [retrieved on 2019-08-13] * page 1 - page 4 * * page 10 * * page 25 - page 26 * | 1-15 | TECHNICAL FIELDS SEARCHED (IPC) G06N G06K G06T A61B |

-/--

The present search report has been drawn up for all claims

| Place of search | Date of completion of the search | Examiner |
|---|---|---|
| The Hague | 2 March 2020 | Bohn, Patrice |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

| | Europäisches Patentamt<br>European Patent Office<br>Office européen des brevets | **EUROPEAN SEARCH REPORT** | Application Number<br>EP 19 19 5868 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | SERIZEL ROMAIN ET AL: "Mini-batch stochastic approaches for accelerated multiplicative updates in nonnegative matrix factorisation with beta-divergence",<br>2016 IEEE 26TH INTERNATIONAL WORKSHOP ON MACHINE LEARNING FOR SIGNAL PROCESSING (MLSP), IEEE,<br>13 September 2016 (2016-09-13), pages 1-6, XP032996274,<br>DOI: 10.1109/MLSP.2016.7738818<br>[retrieved on 2016-11-08]<br>* paragraph [04.3] *<br>----- | 7 | |

**TECHNICAL FIELDS SEARCHED (IPC)**

The present search report has been drawn up for all claims

| Place of search | Date of completion of the search | Examiner |
|---|---|---|
| The Hague | 2 March 2020 | Bohn, Patrice |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **RUFF, L. ; VANDERMEULEN, R. ; GOERNITZ, N. ; DEECKE, L. ; SIDDIQUI, S.A. ; BINDER, A. ; MULLER, E. ; KLOFT, M.** Deep one-class classification. *ICML,* 2018, vol. 80, 4393-4402

- **KINGMA, D.P. ; BA, J.** Adam: A method for stochastic optimization. *ICLR,* 2014